# EUROPEAN PATENT SPECIFICATION

(11) **EP 3 930 803 B1**
(45) Date of publication and mention of the grant of the patent: **09.04.2025**
(21) Application number: 20705750.6
(22) Date of filing: 25.02.2020
(51) Int. Cl.: A61M 5/32

(54) **NEEDLE COVER FOR A MEDICAL INJECTION DEVICE**
NADELABDECKUNG FÜR EINE MEDIZINISCHE INJEKTIONSVORRICHTUNG
ÉTUI PROTECTEUR D'AIGUILLE POUR DISPOSITIF D'INJECTION MÉDICALE

(30) Priority: 26.02.2019 EP 19305222
(43) Date of publication of application: 05.01.2022
(73) Proprietor: Becton Dickinson France, 38800 Le Pont de Claix (FR)
(72) Inventor: HUANG, Longxiang, Jiangsu, Suzhou (CN); YAN, Bo, Shanghai 200126 (CN); ZUCCHELLI, Jéremy, 38400 Saint Martin D'Heres (FR)
(74) Representative: Regimbeau
(86) International application number: PCT/EP2020/054901
(87) International publication number: WO 2020/173937

(56) References cited:
- EP-A1- 2 238 999
- WO-A1-2008/086004
- WO-A1-2012/023938
- WO-A1-2017/089265

## Description

### TECHNICAL FIELD OF THE INVENTION

The invention relates to a needle cover adapted to be mounted on a tip of a medical injection device for covering a needle attached thereon. The invention also relates to a medical assembly comprising a medical injection device and a needle cover for enclosing the needle of the medical injection device.

### TECHNICAL BACKGROUND

Medical injection devices such as syringes typically include a container for containing a medical composition. Said container of said medical injection devices usually comprises an end piece in a form of a longitudinal tip defining a fluid path through which the medical solution is expelled from the container and/or reservoir. A needle is attached to the tip in order to prick the patient's skin and to perform the injection of the composition.

In order to prevent any injury prior to final use, a needle cover is mounted on the tip so as to enclose the needle. This renders the needle physically inaccessible by the persons around the device. The needle cover comprises an inner needle shield, in a material with elastomeric properties, and may further comprise an outer needle shield, in rigid plastic, surrounding the inner needle shield.

The inner needle shield ensures the sealing of the medical injection device. To that purpose, the inner needle shield comprises a sealing portion that sealingly contacts the outer surface of the syringe's tip to provide a tight seal. The inner needle shield prevents any contamination of the medical composition from the outside environment, thereby assuring the container closure integrity. The inner needle shield further prevents any leakage of composition from the outlet of the needle to the external environment. To that purpose, the needle is preferably pricked in the inner needle shield.

The needle cover may be removed from the medical injection device tip shortly prior to the use of the medical injection device.

A drawback of the known needle covers is that it may be relatively difficult to remove them from the tip. In order to do so, the user has to grip both the injection device and the needle cover, and to pull the needle cover. Pulling the needle cover requires the user to exert a quite important effort.

The force needed to remove a needle cover is measured by a physical parameter called "pull out force" (acronym POF). The pull out force necessary for removing the known needle covers from an injection device, such as a syringe, may be quite high and is due in particular to the pressure exerted onto the tip by the inner needle shield which results in friction between the inner needle shield and the tip.

As a consequence, a user having a reduced strength, for example weakened by a disease, may not be able to remove the needle shield and use the injection device for his treatment.

Moreover, healthcare professionals who often use injection devices, such as nurses, have a high risk of injuring themselves, since they may not control the force they apply for pulling off the needle cover from the injection device, which may result in uncontrolled and dangerous movements. Lastly, the needle of the syringe may be bent during the removing of the needle shield because of this high required pull out force.

The patent application WO 2012/023938 A1 teaches an alternative method for reducing the pullout force for removing a needle shield of a syringe.

### BRIEF DESCRIPTION OF THE INVENTION

The invention aims to provide a needle cover for a medical injection device that allows reducing the pull out force while still providing a tight sealing with the tip of the medical injection device.

To this end, one object of the invention is a needle cover for protecting a needle mounted on a tip of a barrel of a medical injection device, wherein the tip extends from a distal face of the barrel, the needle cover comprising:
- an inner needle shield extending along a longitudinal axis, comprising an inner proximal connection part configured to sealingly contact the tip of the barrel,
- an outer needle shield surrounding at least partially the inner needle shield, and fixed to said inner needle shield,
the needle cover being mainly characterized in that it comprises at least one actuator integral with the outer needle shield, said at least one actuator comprising a proximal inner surface which is tilted relative to the longitudinal axis, the actuator being radially movable inwardly relative to the longitudinal axis to slidingly engage the proximal inner tilted surface with the distal face of the barrel so as to cause the needle cover to move in a distal direction along the tip by a wedge effect.

In this application, the "distal direction" is to be understood as meaning the direction of injection. The distal direction corresponds to the travel direction of the plunger rod during the injection, the medical composition contained initially in the barrel being expelled from the latter. The "proximal direction" is to be understood as meaning the opposite direction to said direction of injection.

By "wedge effect" is meant in the present text the conversion, by the tilted proximal inner surface, of a radial force exerted by a user onto the actuator into an axial force which facilitates removal of the needle cover.

According to other optional features of the connector of the invention:
- the needle cover comprises:
   - an axial clearance, which is spared between the needle cover and the distal face of the barrel when said needle cover is mounted on the tip of the barrel, and
   - the actuator comprises at least one lug projecting radially inwardly, said lug comprising said proximal inner tilted surface,
      wherein the lug is configured to be inserted in the axial clearance when the proximal inner tilted surface engages the distal face of the barrel, to abut the needle cover and to push said needle cover in the distal direction;
   - the proximal inner surface is tilted at an angle comprised between 40° and 50° relative to the longitudinal axis;
   - the actuator is a flexible actuator configured to deform radially inwardly relative to the longitudinal axis;
   - the flexible actuator is configured to deform elastically;
   - the actuator comprises one or more flexible arms extending radially from the needle shield in the proximal direction, each arm being radially movable inwardly relative to the longitudinal axis;
   - the actuator comprises two flexible arms, said arms being opposite with respect to the longitudinal axis;
   - the inner needle shield is made in a material with elastomeric properties;
   - the material with elastomeric properties is a thermoplastic elastomer, an elastomer, or a rubber;
   - the outer needle shield is made in a rigid material;
   - the outer needle shield is made in rigid plastic.

Another object of the invention is a medical assembly comprising:
- a medical injection device comprising:
   - a barrel adapted to contain a medical composition,
   - a tip extending from a distal face of the barrel, defining a fluid path extending through the tip and in fluid communication with the barrel,
   - a needle attached to the tip and in fluid communication with the fluid path,
- a needle cover as described previously, mounted on the tip of the medical injection device.

The barrel and the tip of the medical injection device are preferably made of glass.

The medical assembly may comprise an axial clearance between the needle cover and the distal face of the barrel, the proximal inner tilted surface facing said axial clearance.

Another object of the invention is a method for removing a needle cover as described previously, from the tip of a medical injection device, comprising the following steps:
- pinch the actuator radially inwardly to slidingly engage the tilted surface with the distal face of the barrel, so as to cause the needle cover to move in a distal direction along the tip by a wedge effect,
- pull the needle cover in the distal direction relative to the barrel to remove the needle cover from the tip of the injection device.

Advantageously, said pinching and pulling may be carried out in a single step, with the user's fingers located in a same position. Preferably, the user's fingers are positioned around the tilted surface to maximize the wedge effect.

### BRIEF DESCRIPTION OF THE DRAWINGS

Further features and advantages of the invention will become apparent from the detailed description to follow, with reference to the appended drawings, in which:
figure 1 is a side view of an embodiment of a medical assembly of the invention, comprising a needle cover and an injection device,
figure 2 is a side view of a medical injection device without a needle cover attached thereon,
figure 3 is a perspective view of an embodiment of the needle cover of the invention,
figure 4 illustrates a front view and a side view of the needle cover of figure 3,
figure 5 is a side view of a medical assembly of the prior art, that illustrates the removal of the needle cover from the tip of the injection device,
figure 6 is a side view of a medical assembly of the invention, that illustrates the removal of the needle cover from the tip of the injection device before pinching the needle cover,
figure 7 is a side view of the medical assembly of figure 4, that illustrates the removal of the needle cover from the tip of the injection device during pinching the needle cover,
figure 8 is a comparative graph illustrating the evolution of the force exerted onto the needle cover for removing it from the tip of an injection device, in function of the course of the needle cover, for a needle cover of the prior art and a needle cover of the invention,
figure 9 is a graph illustrating a comparison of the pull out force values corresponding to the graph of figure 8.

### DETAILED DESCRIPTION OF EMBODIMENTS OF THE INVENTION

The invention relates to a needle cover 1 configured to be sealingly mounted to the tip 42 of a medical injection device 40 provided with a needle 45, so as to protect the needle.

When the needle cover 1 is attached to the injection device 40, the combination of the needle cover and the injection device forms a medical assembly 100 that prevents a user from contacting the needle enclosed in the needle cover, while protecting the needle from any external contamination.

The assembly 100 is stored before use, meaning before injection of the composition contained in the barrel to a patient.

The medical injection device 40 is preferably a syringe.

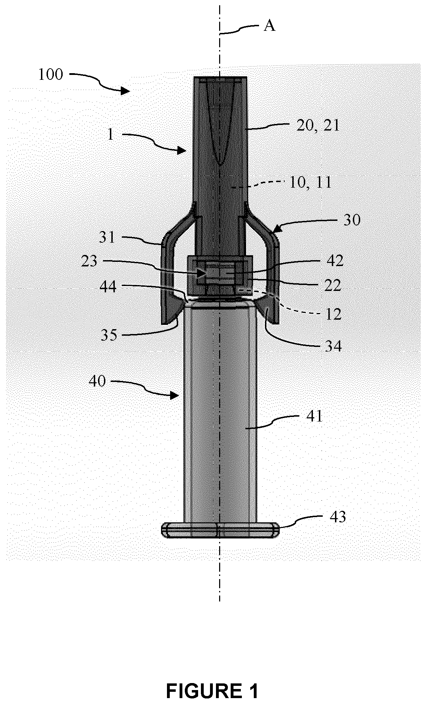

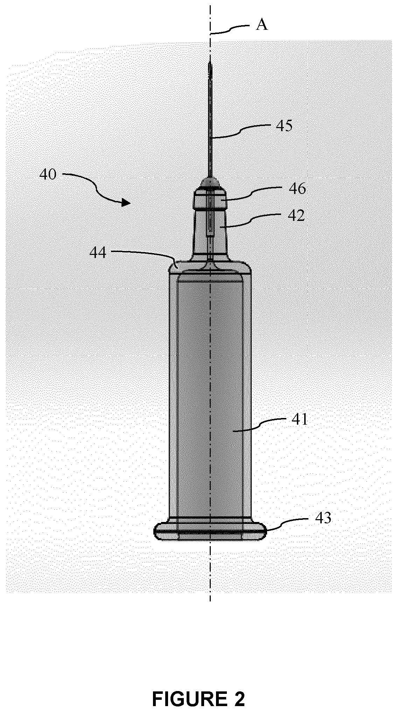

As illustrated in figures 1 and 2, the medical injection device 40 comprises a barrel 41 extending along a longitudinal axis A from a proximal face 43 to a distal face 44 and adapted to contain a medical composition to be injected, and a plunger rod (not represented) translationally movable inside the barrel 41 from a proximal position to a distal position for injecting the composition.

The medical injection device 40 further comprises a distal tip 42 extending along the axis A from the distal face 44 of the barrel. The distal tip 42 is at least partially hollow so as to form a fluid path in fluidic communication with the barrel.

A needle 45 is attached to the tip 42 of the injection device and is in fluid communication with the fluid path.

The medical injection device 40 is preferably made of glass, and more preferably is a glass syringe. Such glass syringes are largely used in hospital environment and readily sterilizable. The medical injection device is preferably a prefilled syringe. The medical injection device is more preferably a syringe with a staked needle.

The needle cover 1 comprises an inner needle shield 10 provided with a body 11 that extends along a longitudinal axis B. The axis B coincides with the axis A when the needle cover is mounted on the tip of the injection device. The needle cover 1 further comprises an outer needle shield 20 configured to surround at least partially the inner needle shield 10, thereby at least partially enclosing said inner needle shield.

The outer needle shield 20 comprises at least one proximal actuator 30.

The actuator is preferably integral with the outer needle shield, i.e. formed of the same material, in one piece (not requiring any assembly). For example, if the outer needle shield is made by injection molding, the actuator is formed using the same step of injection molding.

The actuator is preferably integral with the outer needle shield by the distal part of the actuator.

The outer needle shield 20 comprises at least one lug 34 that project radially inwardly. The lug 34 advantageously flares inwardly, thereby defining a first inner surface 35 oriented proximally and a second surface 36 oriented distally, up to a pointy end.

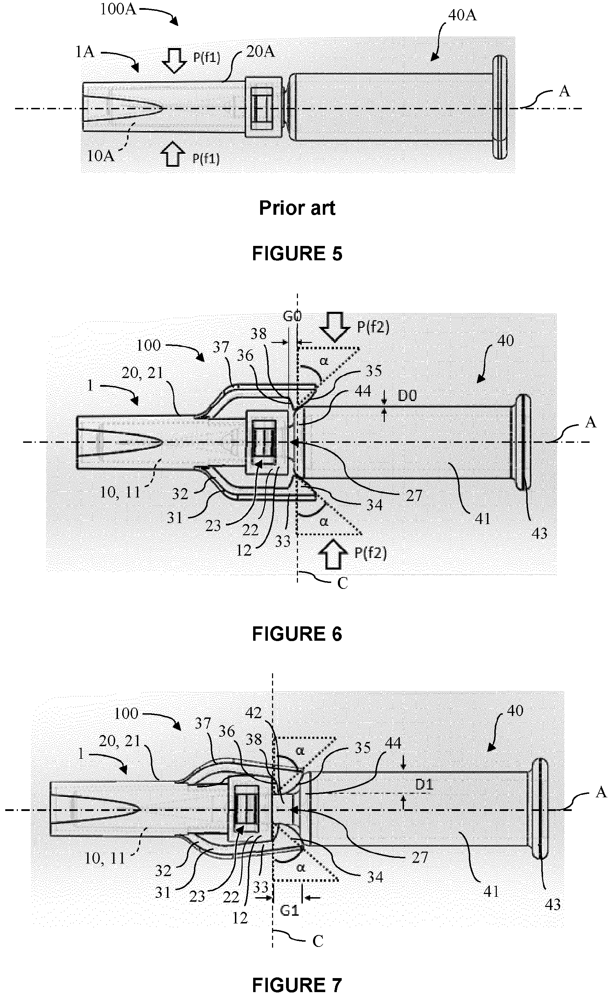

In reference to figure 6, the needle cover 1 at least partially encloses the tip 42 of the injection device, thereby sparing an axial clearance G₀ between the needle cover 1 and the distal face 44 of the barrel. This means that the needle cover 1 does not abut onto the distal face 44 of the barrel.

The axial clearance G₀ advantageously acts as a housing 27 configured to accommodate the lugs 34.

The first inner surface 35 is advantageously curved, the curvature being oriented inwardly towards the axis B.

The first inner surface 35 is tilted relative to the axis B. In other terms, the first inner surface 35 and the axis B are not parallel. As such, the first inner surface is also tilted relative to an axis C perpendicular to the axis A, that extends along the distal face of the barrel (see Figures 6 and 7). In the following of the present text, the first inner surface 35 will be called "tilted surface". The angle between the tilted surface and the axis C, referred to as "angle α", is preferably comprised between 40° and 50°. The angle between the tilted surface and the axis B, equals to 90° minus α, is preferably comprised between 40° and 50°. Since the barrel extends parallel to the axis B, the angle between the tilted surface and the axis B is also the angle between the tilted surface of the arms and the barrel.

According to a preferred embodiment, the proximal actuator 30 comprises one or more flexible arms 31.

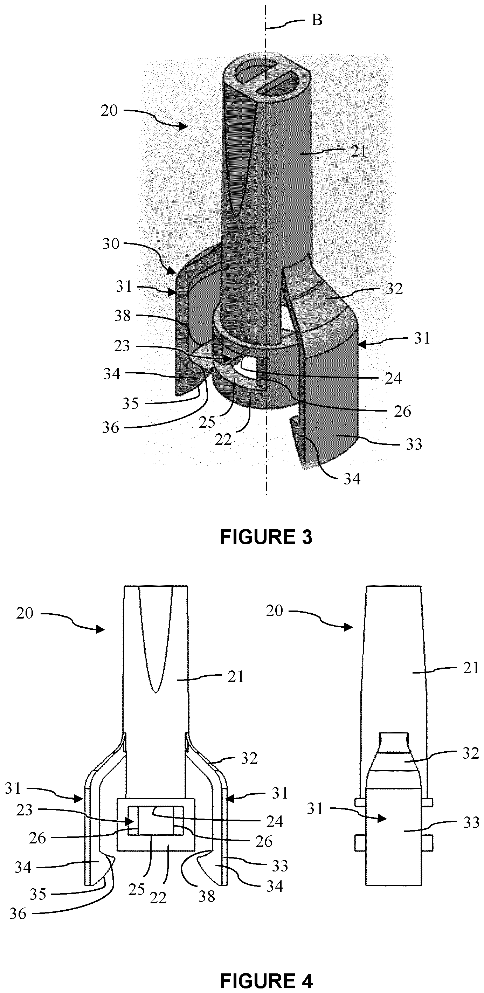

In the embodiment represented in figures 3 and 4, the outer needle shield 20 comprises two flexible arms 31, which are opposite with respect to the axis B. Alternatively, the outer needle shield may comprise only one flexible arm.

Each flexible arm 31 extends from the body 21 of the outer needle shield in the proximal direction.

Each flexible arm 31 advantageously comprises a first portion 32 that extends radially outwardly from the body 21 of the outer needle shield in the proximal direction, and a second portion 33 joined at a junction 37 to the first portion that further extends in the proximal direction.

In the represented embodiments, the flexible arms 31 extend radially outwardly from the outer needle shield. Hence, when no effort is exerted thereon, the flexible arms 31 are remote from the barrel of the injection device, and in particular from the distal face of the barrel, as particularly visible in figures 1 and 6. The distance between the arms 31 and the barrel 41 may of course be adjusted. This configuration of the flexible arms is required when the diameter of the outer needle shield is equal or less than the diameter of the barrel, in order to spare a predetermined distance between the flexible arms and the barrel.

When the outer needle shield 20 comprises at least one arm 31, the lug 34 projects radially inwardly from the arm.

When the outer needle shield 20 comprises arms 31 which are in the actuation position, the axial clearance G₀ accommodate the lugs 34 of said arms 31.

Alternatively, when the diameter of the outer needle shield 20 is greater than the diameter of the barrel 41, the flexible arms 31 may simply extend longitudinally without flaring outwardly, since a distance between the arms and the barrel is already spared.

Each flexible arm 31 is radially movable inwardly relative to the longitudinal axis B in response to an effort oriented radially inwardly applied onto said arm.

In more details, said arm 31 is movable between a rest position and an actuation position wherein the tilted surface engages the distal face 44 of the barrel 41. Preferably, in the rest position, the second portion 33 of the arm is remote from the barrel 41 of a predetermined distance. The engagement of the tilted surface 35 with the distal face 44 of the barrel causes the needle cover 1 to move in a distal direction along the tip 42 by a wedge effect. This aspect will be described in more details in the following.

Preferably, the flexible arm 31 is configured to deform elastically. In other terms, the flexible arm can move back from the actuation position to the rest position with no intervention of the user.

According to a preferred embodiment, the needle cover comprises two arms which are symmetrical with respect to the axis B. This means that the arms have the same dimensions, are arranged similarly relative to the body of the needle cover, and define the same angle α with the axis C.

The dimensions of the arms 31 may be adjusted in order to provide a good flexibility, providing a sufficient wedge effect, and allowing insertion of the lugs 34 into the housing 27 between the needle cover and the injection device. For information, the ratio of the length of a lug on the total length of an arm is comprised between 0.2 and 0.5. Also, a ratio of the ratio of the thickness (or height) of a lug on the total thickness of an arm including the lug is comprised between 0.5 and 0.75.

The body of the inner needle shield preferably has a cylindrical shape and a circular cross section.

The body of the inner needle shield comprises an inner proximal connection part 12 configured to sealingly engage the tip 42 of the injection device. The inner proximal connection part 12 has preferably a larger diameter that the rest of the body. The inner proximal connection part is thus easier to distinguish from the rest of the body and the fixation of the inner needle shield 10 to an outer needle shield 20 is improved as described in the following.

When the needle cover 1 is mounted on the injection device 40, the inner needle shield 10 encloses at least a portion of the tip 42, and the proximal connection part firmly contacts a proximal portion of the tip. Advantageously, the inner needle shield 10 encloses at least the outer surface of the bulge 46. The needle cover is thus tightly and sealingly connected to the tip. The inner needle shield 10 is preferably made in a material with elastomeric properties. In this way, the proximal connection part may slightly deform when connecting the inner needle shield to the injection device so as to match the shape of the tip. Meanwhile, the needle tip may penetrate the inner needle shield. This further reduces the risk of leakage of the medical composition via the needle to the external environment.

The material with elastomeric properties is preferably a thermoplastic elastomer, an elastomer, or a rubber. Preferably, the material with elastomeric properties is sterilizable.

The outer needle shield 20 is preferably made in a rigid material. Preferably, the rigid material is rigid plastic. The rigid material confers rigidity to the outer needle shield, which allows said outer needle shield to better protect the needle shield from shocks. The structural integrity of the needle cover is thereby improved.

An embodiment of the outer needle shield is illustrated in figures 3 and 4.

According to this embodiment, the outer needle shield 20 comprises a body 21 that extends along the longitudinal axis B. The body preferably has a cylindrical shape.

The body 21 comprises a proximal portion 22 that surrounds the inner proximal connection part 12 of the inner needle shield.

The proximal portion 22 has preferably a larger diameter than the rest of the body 21, for enclosing the inner proximal connection part 12 of the inner needle shield.

The outer needle shield 20 is fixed to the inner needle shield 10. To this end, the proximal portion 22 of the outer needle shield is advantageously provided with a through notch 23 comprising an upper part 24, a lower part 25 and side parts 26; and that at least partially extends along the circumference of the proximal portion. Hence, the inner proximal connection part 12 of the inner needle shield is partially inserted in the through notch 23, in a snap-fit connection. The inner proximal connection part 12 of the inner needle shield abuts both the upper part 24 and the lower part 25 of the notch, thereby preventing any translational movement of the inner shield along the axis B relative to the outer needle shield.

Advantageously, the inner proximal connection part 12 also abuts the side parts 26 of the notch 23, thereby preventing any rotational movement of the inner shield around the axis B relative to the outer needle shield.

The through notch 23 is also advantageous as it forms a window for the user, who can make sure that the inner needle shield 10 is enclosed in the outer needle shield 20.

The inner needle shield 10 and the outer needle shield 20 may be fixed together by other fixing means than the snap-fit connection of the notch 23 and the inner proximal connection part 12, or in addition to said snap-fit connection. Alternatively, the outer surface of the inner needle shield and the inner surface of the outer needle shield may comprise structural elements having matching shapes.

The functioning of the medical assembly 100 of the invention will now be described in reference to figures 5, 6, and 7.

Figure 5 illustrates a medical assembly 100A of the prior art. In order to remove the needle cover 1A from the medical injection device 40A, the user first presses or pinches the outer needle shield 20A from both sides, thereby exerting an effort oriented radially inwardly onto an intermediate portion of the outer needle shield. The effort exerted by the user, called "pinch force", is noted P(f₁).

This effort deforms the inner needle shield 10A. The intermediate portion of the inner needle shield is pressed against the tip whereas the inner proximal connection part slightly comes off from the tip. This leads to a modification of the pressure distribution over the contact interface between the inner surface of the inner needle shield and the outer surface of the tip.

The user then pulls the needle cover 1A in the distal direction relative to the barrel 41A, while still pinching the needle cover 1, to remove said needle cover from the tip of the injection device.

Despite achieving the removal of the needle cover 1A from the injection device 40A, the pressure exerted onto the tip by the inner needle shield is high. Indeed, the force exerted by the user to pinch the needle cover 1A is high and causes friction between the inner needle shield and the tip. Such friction limits translational movement of the needle cover 1A relative to the tip 42A, which results in a high pull out force.

Figures 6 and 7 illustrate a medical assembly 100 according to the invention, wherein two arms 31 of the actuator are in the rest position and in the actuation position respectively.

In reference to figure 6, an axial clearance noted G₀ is spared between the needle cover 1 and the distal face 44 of the barrel 41, thereby defining a housing 27. The tilted surface 35 of each arm frictionally engages the distal face 44 of the barrel, and defines an angle α with the axis C. Note that alternatively, the arms may be remote from the barrel in the rest position.

In order to remove the needle cover 1 from the medical injection device 40, the user first pinches the two arms, thereby exerting an effort oriented radially inwardly onto said arms. The pinch force is noted P(f₂). Preferably, the pinching effort is exerted around the axial clearance.

This effort causes the arms to move radially inwardly from the rest position to the actuation position as illustrated in figure 7.

The junction 37 between the first portion 32 and the second portion 33 of each arm bends heading radially outwardly.

The tilted surface 35 of each arm slides over the distal face 44 of the barrel as the lugs 34 get into the housing. This aspect will be described in more details in the following.

The sliding of the tilted surface 35 of the arms over the distal face 44 of the barrel causes a force transfer from the radial direction to the distal direction by a wedge effect. In other terms, the pinch force is converted into a translational force towards the distal direction.

This causes the needle cover 1 to be moved of a determined distance, noted Δx, in the distal direction by the wedge effect, with no pull out force exerted by the user. The distance between the position of the needle cover moved of Δx and the distal face of the barrel is noted G₁. As such, Δx = G₁ - G₀.

The determined distance Δx depends on the angle α. Indeed, the greater the angle α, the greater the distance Δx. However, the greater the angle α, the greater the pinch force as well. Therefore, a balance shall be found between the desired distance Δx and the maximum pinch force required, while keeping in mind that a high pinch force required to remove the needle cover may be detrimental to the user.

The user then pulls the needle cover 1 in the distal direction relative to the barrel 41, while still pinching the arms, to remove said needle cover from the tip 42 of the injection device.

The distance that the needle cover has to travel for being removed from the tip of the injection device, when the user pulls said needle cover, is thus reduced by the distance Δx.

Moreover, compared to the medical assembly 100A of the prior art, the user does not pinch the body of the needle cover, but the arms 31 of the actuator. Hence, the inner surface 35 of the inner needle shield is not pressed against the outer surface of the tip 42, and resulting friction between these two surfaces is thus prevented.

The wedge effect provided by the sliding of the tilted surface 35 over the distal face 44 of the barrel, and the pinching force exerted onto the arms 31 instead of the body of the needle cover 1A, strongly reduces the pull out force. The pull out force is reduced by about 30% to 35% (see examples below).

According to the embodiment illustrated in figures 6 and 7, the lugs 34 provided on the second part of the arms are configured to insert in the housing 27.

In more details, when the arms 31 are in the rest position, the lugs 34 are remote from the housing 27 and may contact or may be remote from the distal face 44 of the barrel.

When the arms 31 are moved from the rest position to the actuation position, the lugs 34 enter the housing 27 while the tilted surface 35 slides over the distal face 44 of the barrel.

After further pinching of the arms, the lugs 34 contact the proximal portion 22 of the outer needle shield, and preferably the inner proximal connection part of the inner needle shield. In the illustrated embodiment, the base 38 of the lugs, delimited by the slope discontinuity between a lug and the second part of an arm, abuts the periphery of the proximal portion 22 of the outer needle shield.

Further pinching of the arms 31 causes the second inner surface 36 of the lugs 34 to abut the proximal portion 22 of the outer needle shield. The arms 31 thereby push the needle cover 1 in the distal direction. This additional effort adds to the wedge effect, for further moving the needle cover in the distal direction relative to the tip 42 of the injection device, before the user pulls the needle cover 1 to remove it from the injection device. The pull out force is thereby further decreased.

The medical assembly of the invention also lead to a reduction of the pinch force.

Indeed, in the case of the known medical assemblies wherein the user pinches the body of the needle cover, since the outer needle cover is usually made of a rigid material, the pinching force to be applied by the user is high. In particular, the pinching force must be sufficient to deform the outer needle shield, which in turn, exerts an effort onto the inner needle shield so as to allow the needle cover to move in a distal direction relative to the tip when the user further pulls said needle cover.

In the case of the medical assembly of the invention, the user pinches arms that are flexible, which means that such arms are configured to deform radially inwardly under a corresponding effort of the user. The mechanical features of the arms, such as their dimensions and constitutive material in particular, are adjusted for this purpose.

Of course, as already mentioned, the pinch force also depends on the angle α, which may be adjusted, as well as the mechanical features of the arms, so as to achieve reasonable pinch force.

### EXAMPLES: MEASUREMENT OF THE PULL OUT FORCE AND CALCULATION OF THE PINCH FORCE

### Measurement of the pull out force

Needle covers of the prior art (which do not comprise any actuator, such as lug or arm) and needle covers of the invention, both being previously mounted onto the tip of a glass syringe, are compared herein. The glass syringes are identical for the needle covers of the prior art and the needle covers of the invention, and belong to the same production batch.

In these assays, ten needle covers of the prior art and ten needle covers of the invention provided with two flexible arms were assessed.

Measurements of the force needed to remove the needle cover from the syringe are carried out. The test is performed with a traction bench (500 mm/min). The method comprises the steps of:
- placing the syringe on a holder,
- holding the needle cover with pneumatic jaws,
- pulling the needle cover at a constant displacement rate to remove it.

For each needle cover of the invention, the flexible arms are manually pinched to the end of clearance. As a result, the needle cover moved in the distal direction relative to the tip of the syringe of a maximum distance Δx. Then the needle covers are positioned in the traction bench to measure the remaining force to be exerted so as to remove the needle cover from the tip of the syringe.

The needle covers of the prior art are directly positioned in the traction bench.

The force needed to pull the needle cover so as to remove it from the tip is recorded, in function of the displacement of the needle cover and the time.

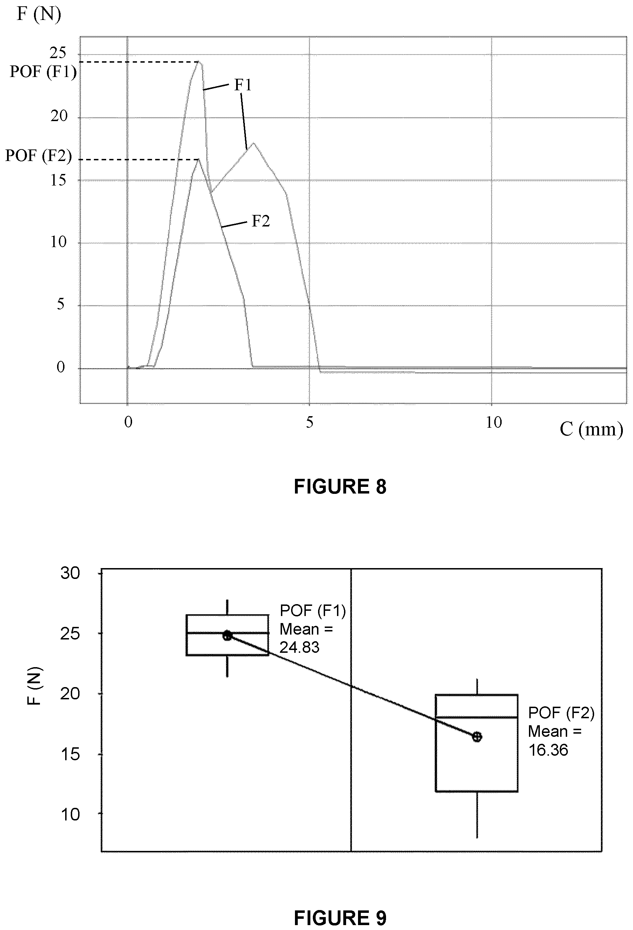

Figure 8 illustrates the evolution of the pulling force F exerted onto the needle cover of the prior art (curve F1) and onto the needle cover of the invention (curve F2) so as to remove it from the syringe tip, relative to the course C of the needle cover. The course C represents the gap between the needle cover and the tip of the injection device. The course of the needle cover of the invention starts after the course of the needle cover of the prior art because the needle cover of the invention was manually pinch press to the end of clearance.

The force needed to remove the needle cover increases at the beginning of the movement of the needle cover, until it reaches a maximum value, named "POF value", that corresponds to the pull out force. As visible on curve F1 of figure 8, the pull out force is about 24 N (Newton). This result is confirmed by figure 9, which represents the average value of the POF measured for all the needle covers of the prior art (POF (F1)) and the average value of the POF measured for the needle covers of the invention (POF (F2)). The mean value of the POF measured for the needle covers of the prior art is 24.83 N. On curve F1 of figure 8, the force then increases again in a second peak which is due to the slip-stick effect resulting from the sliding of the needle cover relative to the syringe tip.

The curve F2 illustrates the evolution of the pulling force F exerted onto the needle cover of the invention so as to remove it from the syringe tip, relative to the course C of the needle cover. The force defines only one peak. Indeed, the first peak visible on curve F1 of figure 8 is absent. This is because the pinching of the flexible arms before the measure already moved the needle cover relative to the syringe tip. As such, part of the force needed to pull the needle cover so as to remove it from the tip is converted into pinch force exerted onto the flexible arms prior to pulling the needle cover. As a result, the pull out force is greatly reduced to about 16 N. This result is confirmed by figure 9, which shows that the mean value of the pull out force for the needle covers of the invention is 16.36 N.

These two values of the pull out force clearly show that the needle cover of the invention leads to a strong decrease of the pull out force compared to the known needle covers. The pull out force reduction is about 34%.

## Claims

1. Needle cover (1) for protecting a needle (45) mounted on a tip (42) of a barrel (41) of a medical injection device (40), wherein the tip (42) extends from a distal face (44) of the barrel, the needle cover (1) comprising:
- an inner needle shield (10) extending along a longitudinal axis (B), comprising an inner proximal connection part (12) configured to sealingly contact the tip (42) of the barrel (41),
- an outer needle shield (20) surrounding at least partially the inner needle shield (10), and fixed to said inner needle shield,
the needle cover (1) being **characterized in that** it comprises at least one actuator (30) integral with the outer needle shield (20), said at least one actuator comprising a proximal inner surface (35) which is tilted relative to the longitudinal axis (B),
the actuator (30) being radially movable inwardly relative to the longitudinal axis (B) to slidingly engage the proximal inner tilted surface (35) with the distal face (44) of the barrel (41) so as to cause the needle cover (1) to move in a distal direction along the tip (42) by a wedge effect.

2. Needle cover according to claim 1, wherein:
- an axial clearance is spared between the needle cover (1) and the distal face (44) of the barrel (41) when said needle cover is mounted on the tip of the barrel, and
- the actuator comprises at least one lug projecting radially inwardly, said lug comprising said proximal inner tilted surface,
wherein the lug is configured to be inserted in the axial clearance when the proximal inner tilted surface engages the distal face of the barrel, to abut the needle cover and to push said needle cover in the distal direction.

3. Needle cover according to any of claim 1 or claim 2, wherein the proximal inner surface is tilted at an angle comprised between 40° and 50° relative to the longitudinal axis (B).

4. Needle cover according to any of the preceding claims, wherein the actuator is a flexible actuator configured to deform radially inwardly relative to the longitudinal axis.

5. Needle cover according to claim 4, wherein the flexible actuator is configured to deform elastically.

6. Needle cover according to any of claim 4 or claim 5, wherein the actuator comprises one or more flexible arms extending radially from the needle shield in the proximal direction, each arm being radially movable inwardly relative to the longitudinal axis.

7. Needle cover according to claim 6, wherein the actuator comprises two flexible arms, said arms being opposite with respect to the longitudinal axis.

8. Needle cover according to any of the preceding claims, wherein the inner needle shield is made in a material with elastomeric properties.

9. Needle cover according to claim 8, wherein the material with elastomeric properties is a thermoplastic elastomer, an elastomer, or a rubber.

10. Needle cover according to any of the preceding claims, wherein the outer needle shield is made in a rigid material.

11. Needle cover according to claim 10, wherein the outer needle shield is made in rigid plastic.

12. Medical assembly comprising:
- a medical injection device comprising:
• a barrel adapted to contain a medical composition,
• a tip extending from a distal face of the barrel, defining a fluid path extending through the tip and in fluid communication with the barrel,
• a needle attached to the tip and in fluid communication with the fluid path,
- a needle cover according to any of claims 1 to 11, mounted on the tip of the medical injection device.

13. Medical assembly according to claim 12, wherein the barrel and the tip of the medical injection device are made of glass.

14. Medical assembly according to claim 12 or claim 13, comprising an axial clearance between the needle cover and the distal face of the barrel, the proximal inner tilted surface facing said axial clearance.

15. Method for removing a needle cover according to any of claims 1 to 11, from the tip of a medical injection device, comprising the following steps:
- pinch the actuator radially inwardly to slidingly engage the tilted surface with the distal face of the barrel, so as to cause the needle cover to move in a distal direction along the tip by a wedge effect,
- pull the needle cover in the distal direction relative to the barrel to remove the needle cover from the tip of the injection device.

## Patentansprüche

1. Nadelabdeckung (1) zum Schutz einer Nadel (45), die an einer Spitze (42) eines Zylinders (41) einer medizinischen Injektionsvorrichtung (40) montiert ist, wobei sich die Spitze (42) von einer distalen Fläche (44) des Zylinders erstreckt, wobei die Nadelabdeckung (1) umfasst:
- einen inneren Nadelschutz (10), der sich entlang einer Längsachse (B) erstreckt, umfassend ein inneres proximales Verbindungsteil (12), das so eingerichtet ist, dass es die Spitze (42) des Zylinders (41) dicht berührt,
- einen äußeren Nadelschutz (20), der den inneren Nadelschutz (10) zumindest teilweise umgibt und am inneren Nadelschutz befestigt ist,
wobei die Nadelabdeckung (1) **dadurch gekennzeichnet ist, dass** sie mindestens einen integral mit dem äußeren Nadelschutz (20) verbundenen Aktuator (30) umfasst, wobei dieser mindestens eine Aktuator eine proximale Innenfläche (35) umfasst, die relativ zur Längsachse (B) geneigt ist,
wobei der Aktuator (30) radial relativ zur Längsachse (B) nach innen beweglich ist, um die proximale geneigte Innenfläche (35) gleitend mit der distalen Fläche (44) des Zylinders (41) in Eingriff zu bringen, sodass sich die Nadelabdeckung (1) durch einen Keileffekt entlang der Spitze (42) in distaler Richtung bewegt.

2. Nadelabdeckung nach Anspruch 1, wobei:
- ein Zwischenraum zwischen der Nadelabdeckung (1) und der distalen Fläche (44) des Zylinders (41) erhalten bleibt, wenn die Nadelabdeckung auf der Spitze des Zylinders montiert ist, und
- der Aktuator mindestens eine radial nach innen vorstehende Nase umfasst, wobei die Nase die proximale geneigte Innenfläche umfasst,
wobei die Nase so eingerichtet ist, dass sie in den axialen Zwischenraum eingeführt wird, wenn die proximale geneigte Innenfläche in die distale Fläche des Zylinders eingreift, um die Nadelabdeckung anzulegen und die Nadelabdeckung in die distale Richtung zu drücken.

3. Nadelabdeckung nach einem der Ansprüche 1 oder 2, wobei die proximale Innenfläche in einem Winkel zwischen 40° und 50° relativ zur Längsachse (B) geneigt ist.

4. Nadelabdeckung nach einem der vorhergehenden Ansprüche, wobei der Aktuator ein flexibler Aktuator ist, der so eingerichtet ist, dass er sich relativ zur Längsachse radial nach innen verformt.

5. Nadelabdeckung nach Anspruch 4, wobei der flexible Aktuator so eingerichtet ist, dass er sich elastisch verformt.

6. Nadelabdeckung nach einem der Ansprüche 4 oder 5, wobei der Aktuator einen oder mehrere flexible Arme umfasst, die sich radial vom Nadelschutz in proximaler Richtung erstrecken, wobei jeder Arm radial relativ zur Längsachse nach innen beweglich ist.

7. Nadelabdeckung nach Anspruch 6, wobei der Aktuator zwei flexible Arme umfasst, wobei sich die Arme in Bezug auf die Längsachse gegenüberliegen.

8. Nadelabdeckung nach einem der vorhergehenden Ansprüche, wobei der innere Nadelschutz aus einem Material mit elastomeren Eigenschaften besteht.

9. Nadelabdeckung nach Anspruch 8, wobei das Material mit elastomeren Eigenschaften ein thermoplastisches Elastomer, ein Elastomer oder ein Gummi ist.

10. Nadelabdeckung nach einem der vorhergehenden Ansprüche, wobei der äußere Nadelschutz aus einem steifen Material hergestellt ist.

11. Nadelabdeckung nach Anspruch 10, wobei der äußere Nadelschutz aus steifem Kunststoff hergestellt ist.

12. Medizinische Baugruppe umfassend:
- eine medizinische Injektionsvorrichtung, umfassend:
• einen Zylinder, der an die Aufnahme einer medizinischen Zusammensetzung angepasst ist,
• eine Spitze, die sich von einer distalen Fläche des Zylinders erstreckt und einen Fluidpfad definiert, der sich durch die Spitze erstreckt und in Fluidkommunikation mit dem Zylinder steht,
• eine Nadel, die an der Spitze angebracht ist und in Fluidkommunikation mit dem Fluidpfad steht,
- eine Nadelabdeckung nach einem der Ansprüche 1 bis 11, die an der Spitze der medizinischen Injektionsvorrichtung montiert ist.

13. Medizinische Baugruppe nach Anspruch 12, wobei der Zylinder und die Spitze der medizinischen Injektionsvorrichtung aus Glas hergestellt sind.

14. Medizinische Baugruppe nach Anspruch 12 oder Anspruch 13, umfassend einen axialen Abstand zwischen der Nadelabdeckung und der distalen Fläche des Zylinders, wobei die proximale geneigte Innenfläche dem axialen Zwischenraum zugewandt ist.

15. Verfahren zum Entfernen einer Nadelabdeckung nach einem der Ansprüche 1 bis 11 von der Spitze einer medizinischen Injektionsvorrichtung, umfassend die folgenden Schritte:
- den Aktuator radial nach innen zusammendrücken, um die geneigte Fläche gleitend mit der distalen Fläche des Zylinders in Eingriff zu bringen, so dass sich die Nadelabdeckung durch einen Keileffekt entlang der Spitze in distaler Richtung bewegt,
- die Nadelabdeckung in distaler Richtung zum Zylinder ziehen, um die Nadelabdeckung von der Spitze der Injektionsvorrichtung zu entfernen.

## Revendications

1. Protège-aiguille (1) pour protéger une aiguille (45) montée sur un embout (42) d'un cylindre (41) d'un dispositif médical d'injection (40), dans lequel l'embout (42) s'étend à partir d'une face distale (44) du cylindre, le protège-aiguille (1) comprenant :
- un protecteur d'aiguille intérieur (10) s'étendant le long d'un axe longitudinal (B), comprenant une partie intérieure proximale de connexion (12) configurée pour entrer en contact de manière étanche avec l'embout (42) du cylindre (41),
- un protecteur d'aiguille extérieur (20) entourant au moins partiellement le protecteur d'aiguille intérieur (10), et fixé audit protecteur d'aiguille intérieur,
le protège-aiguille (1) étant **caractérisé en ce qu'**il comprend au moins un actionneur (30) intégré au protecteur d'aiguille extérieur (20), ledit au moins un actionneur comprenant une surface intérieure proximale (35) inclinée par rapport à l'axe longitudinal (B),
l'actionneur (30) étant mobile radialement vers l'intérieur par rapport à l'axe longitudinal (B) pour engager de manière coulissante la surface intérieure proximale inclinée (35) avec la face distale (44) du cylindre (41) de manière à provoquer le déplacement du protège-aiguille (1) dans une direction distale le long de l'embout (42) par effet de coin.

2. Protège-aiguille selon la revendication 1, dans lequel :
- un jeu axial est ménagé entre le protège-aiguille (1) et la face distale (44) du cylindre (41) lorsque ledit protège-aiguille est monté sur l'embout du cylindre, et
- l'actionneur comprend au moins un ergot faisant saillie radialement vers l'intérieur, ledit ergot comprenant la surface intérieure proximale inclinée,
dans lequel l'ergot est configuré pour être inséré dans le jeu axial lorsque la surface intérieure proximale inclinée est en contact avec la face distale du cylindre, pour venir en butée contre le protège-aiguille et pour pousser ledit protège-aiguille dans la direction distale.

3. Protège-aiguille selon l'une des revendications 1 ou 2, dans lequel la surface intérieure proximale est inclinée d'un angle compris entre 40° et 50° par rapport à l'axe longitudinal (B).

4. Protège-aiguille selon l'une des revendications précédentes, dans lequel l'actionneur est un actionneur flexible configuré pour se déformer radialement vers l'intérieur par rapport à l'axe longitudinal.

5. Protège-aiguille selon la revendication 4, dans lequel l'actionneur flexible est configuré pour se déformer de manière élastique.

6. Protège-aiguille selon l'une des revendications 4 ou 5, dans lequel l'actionneur comprend un ou plusieurs bras flexibles s'étendant radialement à partir de la protection de l'aiguille dans la direction proximale, chaque bras étant radialement mobile vers l'intérieur par rapport à l'axe longitudinal.

7. Protège-aiguille selon la revendication 6, dans lequel l'actionneur comprend deux bras flexibles, lesdits bras étant opposés par rapport à l'axe longitudinal.

8. Protège-aiguille selon l'une des revendications précédentes, dans lequel le protecteur d'aiguille intérieur est fabriqué dans un matériau aux propriétés élastomériques.

9. Protège-aiguille selon la revendication 8, dans lequel le matériau aux propriétés élastomériques est un élastomère thermoplastique, un élastomère ou un caoutchouc.

10. Protège-aiguille selon l'une quelconque des revendications précédentes, dans lequel le protecteur d'aiguille extérieur est fabriqué dans un matériau rigide.

11. Protège-aiguille selon la revendication 10, dans lequel le protecteur d'aiguille extérieur est en plastique rigide.

12. Ensemble médical comprenant :
- un dispositif medical d'injection comprenant
• un cylindre destiné à contenir une composition médicale,
• un embout s'étendant à partir d'une face distale du cylindre, définissant un passage fluidique s'étendant à travers la pointe et en communication fluide avec le cylindre,
• une aiguille fixée à l'embout et en communication fluidique avec le cylindre,
- un protège-aiguille selon l'une des revendications 1 à 11, monté sur l'embout du dispositif médical d'injection.

13. Ensemble médical selon la revendication 12, dans lequel le cylindre et l'embout du dispositif médical d'injection sont en verre.

14. Ensemble médical selon la revendication 12 ou la revendication 13, comprenant un jeu axial entre le protège-aiguille et la face distale du cylindre, la surface intérieure proximale inclinée faisant face à ce jeu axial.

15. Procédé de retrait d'un protège-aiguille selon l'une des revendications 1 à 11, de l'embout d'un dispositif médical d'injection, comprenant les étapes suivantes :
- pincer l'actionneur radialement vers l'intérieur pour engager de manière coulissante la surface inclinée avec la face distale du cylindre, de manière à provoquer le déplacement du protège-aiguille dans une direction distale le long de l'embout par un effet de coin,
- tirer le protège-aiguille dans la direction distale par rapport au cylindre pour retirer le protège-aiguille de l'embout du dispositif d'injection.
